(19) Europäisches Patentamt
European Patent Office
Office européen des brevets

(11) Veröffentlichungsnummer: **0 024 472**
**B1**

(12) # EUROPÄISCHE PATENTSCHRIFT

(45) Veröffentlichungstag der Patentschrift: **10.03.82**

(51) Int. Cl.³: **C 11 D 1/722,** C 07 C 41/03, C 07 C 43/13, B 01 D 19/04

(21) Anmeldenummer: **80102266.6**

(22) Anmeldetag: **26.04.80**

(54) **Grenzflächenspannung öliger Phasen gegen Wasser erniedrigende Verbindungen und Verfahren zu deren Herstellung.**

(30) Priorität: **26.06.79 DE 2925628**

(43) Veröffentlichungstag der Anmeldung: **11.03.81 Patentblatt 81/10**

(45) Bekanntmachung des Hinweises auf die Patenterteilung: **10.03.82 Patentblatt 82/10**

(84) Benannte Vertragsstaaten: **AT BE CH FR GB IT LI LU NL SE**

(56) Entgegenhaltungen:
**DE-A-2 318 950**
**DE-A-2 331 014**
**DE-A-2 432 757**
**DE-A-2 448 388**
**DE-A-2 724 349**
**DE-A-2 810 703**
**FR-A-1 063 244**
**US-A-2 677 700**
**US-A-3 240 819**
**US-A-4 070 298**
**US-A-4 077 895**

(73) Patentinhaber: **CHEMISCHE WERKE HÜLS AG, Postfach 1320, D-4370 Marl 1 (DE)**

(72) Erfinder: **Kosswig, Kurt, Dr., Hoechster Strasse 10, D-4370 Marl (DE)**
Erfinder: **Wienhöfer, Ekkehard, Dr., Griesheimer Strasse 12, D-4370 Marl (DE)**

EP 0 024 472 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Grenzflächenspannung öliger Phasen gegen Wasser erniedrigende Verbindungen und Verfahren zu deren Herstellung

Es ist bekannt, daß polyoxethylierte Fettalkohole und Alkylphenole geeignet sind für den Gebrauch als Reinigungsmittel und Emulgatoren. Bei manchen Anwendungsgebieten, wie z. B. dem Einsatz in Geschirrspül- und Waschmaschinen, stört allerdings das starke Schäumvermögen dieser Substanzen. Man hat daher versucht, durch Zusatz geeigneter Komponenten, wie z. B. höherer Alkohole oder von Blockpolymerisaten aus Polypropylenglykol und Ethylenoxid, dieser unerwünschten Eigenschaft gegenzusteuern.

Ein anderer Weg, der zu dem angestrebten Ziel führen soll, ist die Modifikation des hydrophilen Teils des Tensidmoleküls. Der Ersatz des Wasserstoffs der endständigen Hydroxylgruppe durch einen hydrophoben Rest bewirkt — bei allerdings auch verringerter Wasserlöslichkeit der Substanz — eine deutlich verminderte Schäumneigung im Vergleich zum nicht »blockierten« Ausgangsstoff. Gänzliches Fehlen einer endständigen Hydroxylgruppe führt zusätzlich dazu, daß die betreffenden Substanzen eine verbesserte Stabilität in Anwesenheit von Alkalihydroxiden, -silikaten oder -phosphaten aufweisen, also Stoffen, in Mischung mit denen die fraglichen Tenside etwa in pulverförmigen Geschirrspülmitteln verwendet werden. Die Literatur nennt eine Vielzahl an geeigneten Blockierungsgruppen (N. Schönfeldt, Surface Active Ethylene Oxide Adducts, Pergamon Press, Oxford, 1969, Seite 659 ff.). Häufig erwähnt werden besonders Propylenoxid und Butylenoxid, aber auch Styroloxid und Benzylchlorid. Zu den die Alkalistabilität begünstigenden hydrophoben Resten zählen insbesondere die Isopropyl- und die tert.-Butylgruppen, die durch sauer katalysierte Addition von Propen (DE-B-2 544 569) bzw. Isobuten (DE-B-2 556 499) an die endständige Hydroxylfunktion der genannten Tenside zu erhalten sind. In der US-A-2 903 485 wird das Anlagerungsprodukt eines nicht genau definierten Epoxioctans (»octylene oxide«) an oxethyliertes Alkylphenol im molaren Verhältnis von 1 : 1 als schaumarmes Reinigungsmittel, insbesondere für Geschirrspülmaschinen, beansprucht.

Überraschend wurde nun gefunden, daß demgegenüber die zu der in den Ansprüchen 1 und 2 beschriebenen Stoffklasse gehörenden Verbindungen völlig unerwartete Eigenschaften aufweisen. Hergestellt werden diese durch basisch oder sauer katalysierte Addition von mehr als 1 Mol Alkanepoxid mit 10 bis 22 Kohlenstoffatomen an Oxethylate von Verbindungen mit aktivem Wasserstoff wie Alkylphenole und insbesondere Alkohole des gleichen C-Zahl-Bereichs, wie in den Ansprüchen 3 bis 7 näher erläutert wird. Die Vertreter dieser Stoffklasse sind praktisch nicht mehr wasserlöslich; sie besitzen daher keinen nach den Standardmethoden erfaßbaren Trübungspunkt und können nicht der Klasse schaumarmer Waschrohstoffe zugerechnet werden. Es zeigt sich vielmehr eine ausgeprägte Löslichkeit der neuen Addukte in unpolaren Phasen, wie z. B. Paraffinöl.

Als nicht zu erwartende Eigenschaft der erfindungsgemäßen Verbindungen wurde festgestellt, daß bereits sehr geringe Konzentrationen davon in einer Ölphase deren Grenzflächenspannung gegen Wasser auf Werte $<1$ mN/m erniedrigen. Substanzen, die ein solches Verhalten zeigen, finden als moderierender Zusatz zu stark schäumenden Tensiden Verwendung. Weiterhin sind sie für den Einsatz als Motoröladditiv geeignet, da in Anwesenheit der neuen Verbindungen das Schäumen eines solchen Öls während des Betriebs, das durch eingedrungenes Wasser hervorgerufen wird und die gewünschte Schmierwirkung herabsetzt, unterdrückt wird. Auch an die Verwendung als Emulsionshilfsstoffe ist aufgrund der genannten Eigenschaft zu denken. Die Aufzählung dieser Einsatzgebiete bedeutet natürlich keine Beschränkung, sondern erläutert nur wünschenswerte Vorteile, die man aus dem überraschenden Verhalten der erfindungsgemäßen Verbindungen ziehen kann.

Für das erfindungsgemäße Verfahren geeignete Verbindungen $R(OH)_z$ sind somit solche, an die in Gegenwart eines geeigneten basischen oder sauren Katalysators Ethylenoxid addiert werden kann. In Frage kommen dafür geradkettige und verzweigte, gegebenenfalls substituierte primäre und sekundäre Alkohole, geradkettige und verzweigte 1,2-, 1,3- und $\alpha,\omega$-Alkandiole und Alkylphenole.

Geeignete Stoffe dieser Art sind z. B. 1-Octanol, 2-Octanol, 1-Decanol, 1-Undecanol, 2-Undecanol, 1-Dodecanol, 1-Tridecanol, 2-Tridecanol, 1-Tetradecanol, 1-Hexadecanol, 1-Octadecanol, 1-Eicosanol, 1-Docosanol, 2-Ethylhexanol; Gemische von Alkoholen nativen Ursprungs; Alkohole aus der Aufbaureaktion nach Ziegler und anschließender Oxidation oder deren Gemische sowie die Produkte der Hydroformylierung oder Hydrocarboxymethylierung von end- und innenständigen Olefinen und nachfolgender Hydrierung; Ethylenglykol, Propylenglykol, 1,3-Propandiol, 1,3-Butandiol, 1,4-Butandiol, 1,2-Hexandiol, 1,2-Octandiol, 1,2-Decandiol, 1,2-Tetradecandiol, 1,12-Dodecandiol, 1,2-Hexadecandiol, 1,2-Octadecandiol; Arylalkohole wie 5-Phenyl-1-decanol, 7-Phenyl-1-dodecanol, $C_5-C_{11}$-Phenyl-1-tetradecanol, $C_5-C_{11}$-Phenyl-2-methyl-1-tridecanol; ferner Alkylphenole wie 4-Isononylphenol, 4-Isooctylphenol, 4-n-Dodecylphenol, 4-n-Tridecylphenol oder Gemische der beiden letzteren.

Als erfindungsgemäß geeignete Epoxidkomponenten der Formel

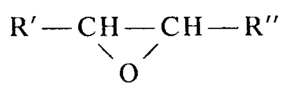

sind 1,2-Epoxide mit 10 bis 22 Kohlenstoffatomen, mittelständige Epoxide der gleichen

Kohlenstoffzahl sowie Gemische daraus und die Epoxidationsprodukte 2-alkylsubstituierter α-Olefine, wie sie bei der alumino-organisch katalysierten Dimerisation kurzkettiger α-Olefine entstehen, anzusehen. Besonders geeignet sind Epoxide mit 10 bis 16 Kohlenstoffatomen.

Solche Epoxide sind z. B. 1,2-Epoxidecan, 1,2-Epoxidodecan, 1,2-Epoxitetradecan, 1,2-Epoxihexadecan, 1,2-Epoxioctadecan, 1,2-Epoxieicosan, 1,2-Epoxi-2-butyloctan, 1,2-Epoxi-2-hexyldecan, 1,2-Epoxidodecan/-tetradecan/-hexadecan als Gemisch; das Produkt der Umsetzung eines Gemisches von mittelständigen Olefinen mit 11 bis 15 Kohlenstoffatomen mit einer geeigneten Percarbonsäure; 1,2-Epoxi-2-ethyldodecan.

Die Mengenverhältnisse von Hydroxikohlenwasserstoff, Ethylenoxid und Epoxid untereinander sollten so beschaffen sein, daß der lipophile gegenüber dem hydrophilen Anteil der nach dem erfindungsgemäßen Verfahren hergestellten neuen Verbindungen die Größenordnung von 35 bis 75, bevorzugt von 40 bis 60 Gewichtsprozent des Moleküls annimmt. Bei Unterschreiten der tieferen Grenze kann die Löslichkeit der Substanzen in der öligen Phase so gering werden, daß an der Phasengrenze eine Abscheidung erfolgt. Als lipophil sind die als Reaktionsstarter eingesetzten Hydroxikohlenwasserstoffe und die Epoxialkane

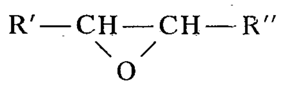

anzusehen, während das aus dem Ethylenoxid stammende Segment wie üblich als hydrophil bewertet wird.

Eine aus 1 Mol 1-Dodecanol, 20 Mol Ethylenoxid und 3 Mol 1,2-Epoxitetradecan zusammengesetzte Verbindung hat somit ein entsprechendes Verhältnis von 48% zu 52%, liegt also im bevorzugten Bereich.

Bei Verwendung der bevorzugten primären Alkohole aus dem Fettalkylbereich $C_8-C_{22}$ als Starter und der aus dem gleichen C-Zahl-Bereich stammenden 1,2-Epoxialkane sowie bei Beachtung des bevorzugten Verhältnisses zwischen lipophilem und hydrophilem Anteil im gewünschten Endprodukt liegen die Molekulargewichte der so erzeugten grenzflächenaktiven Stoffe zwischen 1500 und 2500. Werden für besondere Zwecke spezielle Alkohole oder Epoxide eingesetzt, können die Molekulargewichte natürlich deutlich außerhalb des genannten Bereichs liegen.

Die Umsetzung der Verbindung $R(OH)_z$ mit Ethylenoxid und anschließend mit dem Epoxialkan

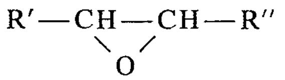

verläuft entweder in Gegenwart eines basischen Katalysators wie Natrium- oder Kaliumhydroxid, Alkoholaten mit 1 bis 4 C-Atomen wie Natriummethylat, Natriumethylat oder Kalium-tert.-butanolat bei Temperaturen von 160 bis 230° C, bevorzugt bei 180 bis 210° C, oder mit sauren Katalysatoren wie Bortrifluorid oder komplexen Oxonium- oder Carbeniumsalzen, beispielsweise Triethyloxonium-tetrafluoborat, bei Temperaturen von 80 bis 160° C, bevorzugt bei 100 bis 140° C. Die eingesetzte Menge an Katalysatoren ist üblich und beträgt 1 bis 3 Gewichtsprozent, bezogen auf den vorgelegten Starter $R(OH)_z$.

Die Umsetzung kann bei Normaldruck ablaufen, jedoch kann vorteilhaft ein Überdruck von 0,5 bis 3 at angewendet werden.

Die erfindungsgemäßen Verbindungen zeigen bereits in sehr geringen Konzentrationen von 0,002 Gewichtsprozent eine gewisse Wirkung; man wird sie jedoch in Konzentrationen von 2 bis 0,02, insbesondere 1 bis 0,2 Gewichtsprozent, anwenden.

Die nun folgenden Beispiele erläutern die Methoden zur Herstellung der neuen Verbindungen und ihre überraschende Wirkung im Vergleich zu modifizierten Ethylenoxid-Additionsverbindungen des Standes der Technik.

## Beispiel 1

62,0 g (0,333 Mol) 1-Dodecanol wurden in Gegenwart von 0,8 g pulverisiertem Natriumhydroxid mit 299,0 g (6,795 Mol) Ethylenoxid bei 160°C umgesetzt. Anschließend wurden 212,3 g (1,000 Mol) 1,2-Epoxitetradecan zugetropft und das Gemisch bei 160°C gerührt. Nach 2 Stunden betrug der Epoxidgehalt 5,2%, nach weiteren 6 Stunden war das Epoxid verbraucht. Die Werte der Grenzflächenspannung sind in Nr. 1 der Tabelle wiedergegeben. Der Polydiolgehalt des Produktes wurde zu 0,1% bestimmt.

## Beispiel 2

72,5 g (0,250 Mol) Alfol 1620 wurden in Gegenwart von 1,0 g pulverisiertem Natriumhydroxid mit 241,0 g (5,477 Mol) Ethylenoxid bei 160°C umgesetzt. Anschließend wurden 117,2 g (0,750 Mol) 1,2-Epoxidecan zugetropft und das Gemisch bei 210°C 2 Stunden gerührt; nach dieser Zeit war das Epoxid verbraucht Die gegenüber Versuch 1) deutlich höhere Temperatur führt also zu stark verkürzten Reaktionszeiten. Der Polydiolgehalt betrug 0,2%. Die Werte der Grenzflächenspannung sind in Nr. 6 der Tabelle wiedergegeben.

## Beispiel 3

46,5 g (0,250 Mol) 1-Dodecanol wurden in Gegenwart von 0,8 g pulverisiertem Natriumhydroxid mit 318 g (7,227 Mol) Ethylenoxid und 106,2 g (0,500 Mol) 1,2-Epoxitetradecan analog zu Beispiel 1 umgesetzt. Das 0,1% Polydiol enthaltende Produkt zeigte in 2- und 0,2%iger Lösung in Paraffinöl an der Grenzfläche zu Wasser kristalline Abscheidungen; die Grenzflächenspannung beider Lösungen lag bei 1 bis 2 mN/m. Der hohe hydrophile Anteil von 68% liegt nicht mehr im bevorzugten Bereich und führt zur Störung des Lösungsverhaltens.

## Beispiel 4

In der nachfolgenden Tabelle 1 sind die Werte der Grenzflächenspannung im Paraffin-Öl/Wasser-System von öligen Lösungen erfindungsgemäßer Tenside bei unterschiedlichen Konzentrationen aufgeführt. Die eingesetzten Produkte wurden aus den genannten Komponenten analog zu Beispiel 2 dargestellt. Zum Vergleich mit angegeben sind die Meßwerte eines Tensids analogen Aufbaus, das jedoch nur ein Mol addiertes 1,2-Epoxialkan enthält, eines tert.-Butylethers, eines oxethylierten Fettalkoholgemisches und eines Ethylenoxid-Propylenoxid-Adduktes an Nonylphenol, das als schaumarmes Tensid in Geschirrspülmitteln Einsatz findet.

## Beispiel 5

37,2 g (0,200 Mol) 1-Dodecanol wurden mit 0,5 ml Bortrifluorid-Etherat versetzt und mit 185 g (4,185 Mol) Ethylenoxid bei 80 bis 90°C umgesetzt. Nach Zugabe von weiteren 0,6 ml Katalysator wurden 127,2 g (0,600 Mol) 1,2-Epoxitetradecan bei 90 bis 100°C zugetropft und das Gemisch anschließend bei 130°C eine Stunde gerührt; ein Gehalt an freiem Epoxid war dann nicht mehr festzustellen. Nach Abziehen von in Nebenreaktionen entstandenen Leichtsiedern verblieb ein farbloses pastöses Produkt, das auf 1 Mol 1-Dodecanol 16,7 Mol Ethylenoxid und 3 Mol 1,2-Epoxitetradecan in Form des linearen Adduktes enthielt. Der Polydiolgehalt betrug 2,4%.

## Beispiele 6 bis 25

Tabelle 2 enthält weitere Beispiele für die erfindungsgemäßen Tenside, die mit unterschiedlichen Ausgangsalkoholen, 1,2-Epoxialkanen und Katalysatoren hergestellt wurden. Alle Produkte sind nicht wasserlöslich und weisen den obengenannten Effekt an Öl/Wasser-Grenzflächen auf.

Tabelle 1

Bei unterschiedlichen Konzentrationen erfindungsgemäßer Tenside in Paraffinöl gegen VE-Wasser gemessene Grenzflächenspannungen bei 20°C

| Nr. | Alkoholkomponente | EO | 1,2-Epoxialkan | Anzahl | Polydiol-gehalt (%) | Grenzflächenspannung (mN/m) bei | | | |
|---|---|---|---|---|---|---|---|---|---|
| | | | | | | 2 % | 0,2 % | 0,02 % | 0,002 % |
| 1 | 1-Dodecanol | 20 | $C_{14}H_{28}O$[a]) | 3 | 0,1 | <1 | <1 | 1,5 | 4 |
| 2 | 1-Dodecanol | 22,6 | $C_{14}H_{28}O$[b]) | 3 | 0,2 | <1 | <1 | <1 | 3,5 |
| 3 | Fettalkohol $C_{10/12}$ | 17,5 | $C_{10}H_{20}O$ | 3 | 0,2 | <1 | <1 | 2 | 4,5 |
| 4 | Fettalkohol $C_{10/12}$ | 21,9 | $C_{14}H_{28}O$ | 3 | 0,3 | <1 | <1 | <1 | 3 |
| 5 | Fettalkohol $C_{10/12}$ | 25 | $C_{16}H_{32}O$ | 3 | 0,5 | <1 | <1 | <1 | 3 |
| 6 | Fettalkohol $C_{16/20}$ | 21,9 | $C_{10}H_{20}O$ | 3 | 0,2 | <1 | <1 | <1 | 5,5 |
| 7 | Fettalkohol $C_{16/20}$ | 31 | $C_{14}H_{28}O$ | 3 | 0,4 | <1 | <1 | <1 | 3 |
| 8 | Fettalkohol $C_{16/20}$ | 31,5 | $C_{16}H_{32}O$ | 3 | 0,4 | <1 | <1 | <1 | 4,5 |
| Vergleich | 1-Dodecanol | 20 | $C_{14}H_{28}O$ | 1 | 0,6 | 1,0 | 1,5 | 3,0 | — |
| Vergleich | Fettalkohol $C_{16/18}$ | 6 | t-$C_4H_9$— | 1 | — | 1,0 | 1,5 | 5,5 | — |
| Vergleich | Nonylphenol | 9 | $C_3H_6O$ | 10 | <3 | 2,5[c]) | — | — | — |

[a]) 1,2-Epoxitetradecan bei 160°C addiert.
[b]) 1,2-Epoxitetradecan bei 210°C addiert.
[c]) 2%ige Lösung VE-Wasser.

Tabelle 2

Erfindungsgemäße Tenside aus unterschiedlichen Startalkoholen, Epoxiden und Katalysatoren

| Beispiel | Alkoholkomponente | EO | 1,2-Epoxialkan | Anzahl | Polydiolgehalt (%) | Katalysator |
|---|---|---|---|---|---|---|
| 6 | Fettalkohol $C_{10/12}$ | 19 | -hexadecan | 3 | 2,3 | $BF_3$ |
| 7 | Fettalkohol $C_{10/12}$ | 14 | -decan | 3 | 1,1 | $BF_3$ |
| 8 | Fettalkohol $C_{16/20}$ | 16 | -decan | 3 | 1,1 | $BF_3$ |
| 9 | Fettalkohol $C_{16/20}$ | 17 | -tetradecan | 3 | 2,1 | $BF_3$ |
| 10 | Fettalkohol $C_{16/20}$ | 20 | -hexadecan | 3 | 1,6 | $BF_3$ |
| 11 | Nonylphenol | 13 | -hexadecan | 1,3 | 0,1 | NaOH |
| 12 | Nonylphenol | 12 | -tetradecan | 1,3 | 0,2 | NaOH |
| 13 | Nonylphenol | 11 | -decan | 1,3 | 0,1 | NaOH |
| 14 | Nonylphenol | 11 | -tetradecan | 2 | 0,1 | NaOH |
| 15 | Nonylphenol | 22 | -tetradecan | 3 | 0,2 | NaOH |
| 16 | Nonylphenol | 16 | -decan | 4 | 0,3 | NaOH |
| 17 | Nonylphenol | 16 | -decan | 2 | 0,4 | NaOH |
| 18 | Nonylphenol | 21 | -decan | 3 | 0,2 | $BF_3$ |
| 19 | Nonylphenol | 20 | -decan | 3 | 0,3 | NaOH |
| 20 | Nonylphenol | 20 | -hexadecan | 3 | 0,4 | NaOH |
| 21 | Nonylphenol | 26 | -hexadecan | 3 | 0,5 | NaOH |
| 22 | 2-Ethylhexanol | 30 | -tetradecan | 3 | 0,4 | NaOH |
| 23 | n-Butanol | 25 | -tetradecan | 3 | 1,2 | NaOH |
| 24 | 1,2-Decandiol | 10 | -decan | 2 | 0,2 | NaOH |
| 25 | 1,4-Butandiol | 13 | -hexadecan | 2 | 0,3 | NaOH |

**Patentansprüche**

1. Zur Erniedrigung der Grenzflächenspannung öliger Phasen gegen Wasser in niedriger Konzentration geeignete Verbindungen der Formel

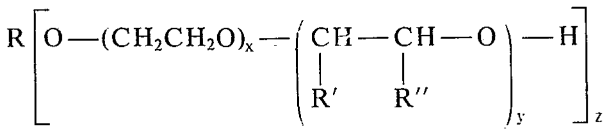

z = 1, 2,

dadurch gekennzeichnet, daß R für z = 1 einen 8 bis 22 C-Atome in der Alkylkette enthaltenden Alkyl-, Aralkyl-, Alkylaryl- oder einen 2 bis 22 C-Atome enthaltenden Hydroxialkylrest und für z = 2 einen 8 bis 22 C-Atome enthaltenden (Aryl-)Alkylidenrest darstellt, R′ und R″ für Wasserstoff oder einen $C_1-C_{20}$-Alkylrest stehen, wobei R′ und R″ nicht gleichzeitig Wasserstoff sind und R′ und R″ zusammen 8 bis 20 Kohlenstoffatome besitzen, x einen Wert von 10 bis 40 und y einen Wert von 1,2 bis 5 annimmt.

2. Zur Erniedrigung der Grenzflächenspannung öliger Phasen gegen Wasser geeignete Verbindungen nach Anspruch 1, dadurch gekennzeichnet, daß x einen Wert von 20 bis 30 und y einen Wert von 1,3 bis 3 annimmt.

3. Verfahren zur Herstellung von Verbindungen nach Anspruch 1, dadurch gekennzeichnet, daß eine Verbindung $R(OH)_z$ in Gegenwart eines basischen oder sauren Katalysators zunächst mit Ethylenoxid und anschließend mit einem Epoxialkan der Formel

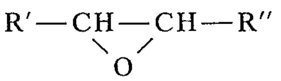

umgesetzt wird.

4. Verfahren nach Anspruch 2, dadurch gekennzeichnet, daß als Starter Fettalkohole mit 8 bis 22 Kohlenstoffatomen oder Gemische davon eingesetzt werden.

5. Verfahren nach Anspruch 2, dadurch gekennzeichnet, daß als Epoxidkomponente 1,2-Epoxialkane des Fettalkylbereichs mit 10 bis 22 Kohlenstoffatomen, oder deren Gemische verwendet werden.

6. Verfahren nach Anspruch 3, dadurch gekennzeichnet, daß bei Verwendung eines basischen Katalysators die Reaktionstemperatur 160 bis 230° C beträgt.

7. Verfahren nach Anspruch 3, dadurch gekennzeichnet, daß bei Verwendung eines sauren Katalysators die Reaktionstemperatur 80 bis 160° C beträgt.

**Claims**

1. Compounds of the general formula

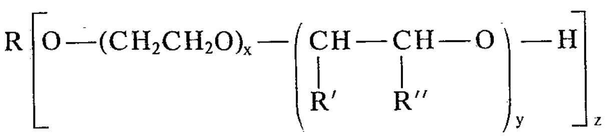

where z = 1 or 2, suitable for use at low concentration to lower the surface tension of oily phases with respect to water, characterised in that when z is 1 R is an alkyl, aralkyl or arylalkyl radical of 8 to 22 carbon atoms in the alkyl chain or a hydroxyalkyl radical of 8 to 22 carbon atoms and when z is 2 R is an optionally aryl-substituted alkylidene radical of 8 to 22 carbon atoms, R′ and R″ are hydrogen or an alkyl radical of 1 to 20 carbon atoms, provided that R′ and R″ are not simultaneously hydrogen and that R′ and R″ together have 8 to 20 carbon atoms, x is a number from 10 to 40 and y is a number from 1.2 to 5.

2. Compounds according to claim 1 suitable for use to lower the surface tension of oily phases with respect to water, characterised in that x is a number from 20 to 30 and y is a number from 1.3 to 3.

3. A process for the manufacture of a compound according to claim 1, characterised in that a compound of the formula $R(OH)_z$ is reacted in the presence of a basic or acidic catalyst firstly with ethylene oxide and then with an epoxyalkane of the formula

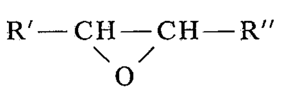

4. A process according to claim 3, characterised in that a fatty alcohol of 8 to 22 carbon atoms or a mixture thereof is used as starting material.

5. A process according to claim 3, characterised in that a 1,2-epoxyalkane in the fatty alkyl range having 10 to 22 carbon atoms or a mixture thereof is used as the epoxide component.

6. A process according to claim 3, characterised in that a reaction temperature of 160 to 230°C is used with a basic catalyst.

7. A process according to claim 3, characterised in that a reaction temperature of 80 to 160° C is used with an acidic catalyst.

**Revendications**

1. Composés appropriés pour abaisser à basse concentration la tension interfaciale de phases huileuses vis-à-vis de l'eau, lesdits composés, qui répondant à la formule

$$R\left[O-(CH_2CH_2O)_x-\left(\begin{array}{l}CH-CH-O\\ |\qquad\ |\\ R'\qquad R''\end{array}\right)_y-H\right]_z$$

dans laquelle z a une valeur égale à l'unité ou à 2, étant caractérisés par le fait que, lorsque z est égal à 1, R représente un reste alcoyle, aralcoyle, alcoylaryle renfermant de 8 à 22 atomes de carbone dans la chaîne alcoyle, ou bien un reste hydroxy-alcoyle renfermant de 2 à 22 atomes de carbone, et représente, lorsque z est égal à 2, un reste (alcoyl-)alcoylidène renfermant de 8 à 22 atomes de carbone, R' et R'' représentent de l'hydrogène ou un reste alcoyle en $C_1$ à $C_{20}$, tandis que R' et R'' ne représentent pas simultanément de l'hydrogène et possèdent ensemble de 8 à 20 atomes de carbone, x a une valeur de 10 à 40 et y a une valeur de 1,2 à 5.

2. Composés appropriés pour abaisser vis-à-vis de l'eau la tension interfaciale de phases huileuses, caractérisés par le fait que x a une valeur de 20 à 30 et y une valeur de 1,3 à 3.

3. Procédé de préparation de composés selon la revendication 1, caractérisé par le fait qu'en présence d'un catalyseur basique ou acide, on fait réagir un composé de formule

$$R(OH)_z$$

d'abord sur de l'oxyde d'éthylène et ensuite sur un époxy-alcane.

4. Procédé selon la revendication 3, caractérisé par le fait qu'on utilise, comme initiateurs, des alcools gras comportant de 8 à 22 atomes de carbone, ou bien des mélange de ceux-ci.

5. Procédé selon la revendication 3, caractérisé par le fait qu'on utilise, comme composant époxyde, des 1,2-époxy-alcanes d'alcools gras comportant de 10 à 22 atomes de carbone, ou leurs mélanges.

6. Procédé selon la revendication 3, caractérisé par le fait que lorsqu'on utilise un catalyseur basique, la température de la réaction est de 160 à 230° C.

7. Procédé selon la revendication 3, caractérisé par le fait que lorsqu'on utilise un catalyseur acide, la température de de réaction est de 80 à 160° C.